# EUROPEAN PATENT APPLICATION

(11) **EP 3 907 740 A1**
(43) Date of publication of application: **10.11.2021**
(21) Application number: 21167619.2
(22) Date of filing: 09.04.2021
(51) Int. Cl.: G16H 30/20, G06F 3/00, G06F 16/00, G16H 40/67

(54) **DATA INTEGRATION SYSTEM**

(30) Priority: 04.05.2020 TW 109205303 U
(71) Applicant: EBM Technologies Incorporated, 114 Taipei City (TW)
(72) Inventor: Pan, William, 104 Taipei City, (TW)
(74) Representative: Straus, Alexander

(57) **Abstract**

A data integration system including an integration device (10) is provided. The integration device receives a first DICOM data object including a first attribute item, searches for a second attribute item corresponding to the first attribute item, and convert the first attribute item of the first DICOM data object to the second attribute item. When the first DICOM data object is transferred from one medical institution (Ha) to another medical institution (Hb), the integration device converts a medical record number that complies with one medical institution organizational architecture into another medical record number that complies with another medical institution organizational architecture to efficiently integrate medical data even if different medical institutions adopt different medical institution organizational architectures.

## Description

### Field of the Invention

The present invention relates to a data integration system, and more particularly, to a data integration system able to integrate medical data of different health facilities (or medical institutions), each of which adopts different organizational architectures.

### Background of the Invention

The picture archiving and communication system (PACS) is a computer system or network system specifically dedicated to storing, obtaining, transmitting and displaying medical images. The PACS may receive images from a variety of medical imaging equipment, such as ultrasound imaging, magnetic resonance imaging (MRI), positron tomography (PT), computed tomography (CT), mammography (MG), digital radiography (DR), Computed radiography (CR), and X-ray plain film (PF).

The digital imaging and communications in medicine (DICOM) protocol used by the PACS is a set of common standard protocols specifically dedicated to processing, storing, printing, and transmitting medical images. The set of common standard protocols regulate definitions of DICOM format files (namely, DICOM data objects) and network communication protocols. The DICOM is based on transmission control protocol (TCP) and internet protocol (IP) (TCP/IP protocol) to communicate between a host (such as a server) and multiple terminals (such as medical instruments or workstations). For example, a workstation and a medical instrument, which are capable of receiving DICOM data objects, may exchange the DICOM data objects (which include medical images and attribute data such as medical record numbers) according to the TCP/IP protocol.

However, different medical institutions usually adopt different medical institution organizational architectures. Therefore, when a DICOM data object is to be transferred from one medical institution to another medical institution, the medical record number in the current attribute data of the DICOM data object does not conform to the organizational architecture of the medical institution in which the DICOM data object is imported, and cannot be used efficiently by different medical institutions with different medical institution organizational architectures. To make use of medical data and promote the progress of medical diagnosis, there is still room for improvement when it comes to a data integration system to integrate medical data especially for different medical institutions of different medical institution organizational architectures.

### Summary of the Invention

It is therefore a primary objective of the present invention to provide a data integration system able to integrate medical data to promote the advancement of medical diagnosis when different health facilities use different organizational architectures.

This is achieved by a data integration system according to the independent claim 1 here below. The dependent claims pertain to corresponding further developments and improvements.

As will be seen more clearly from the detailed description following below, an embodiment of the present invention provides a data integration system, including: an integration device, comprising: a storage circuit, configured to store instructions of: receiving a first digital imaging and communications in medicine (DICOM) data object including a first attribute item; searching for a second attribute item corresponding to the first attribute item; and converting the first attribute item of the first DICOM data object to the second attribute item; and a processing circuit, coupled to the storage device, configured to execute the instructions stored in the storage circuit.

### Brief Description of the Drawings

FIG. 1 is a schematic diagram of an integration device according to an embodiment of the present invention.
FIG. 2 is a schematic diagram of a user interface of a dedicated application software of the integration device shown in FIG. 1 according to an embodiment of the present invention.
FIG. 3 to FIG. 7 are schematic diagrams of data integration systems according to an embodiment of the present invention.

### Detailed Description

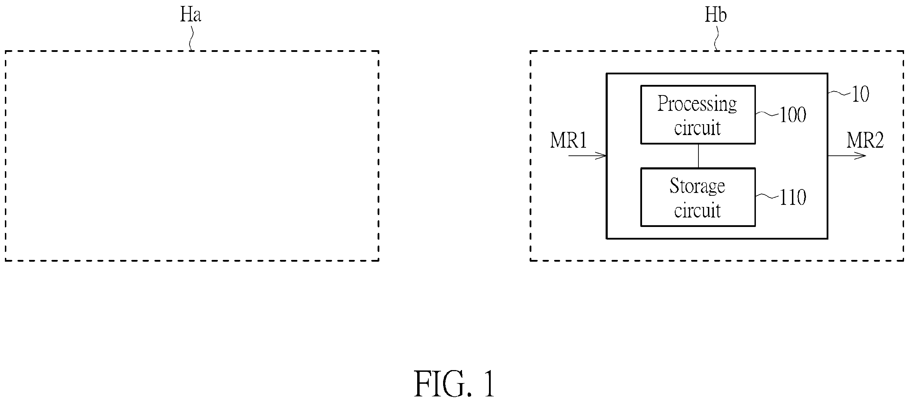

FIG. 1 is a schematic diagram of an integration device 10 according to an embodiment of the present invention. The integration device 10 may be (located) in a medical institution Hb and may be remotely-located from another medical institution Ha. In some embodiments, the integration device 10 may be a (computer) terminal (such as a medical instrument or workstation) or a mobile device (such as a laptop, mobile phone, or tablet), and may include a processing circuit 100 and a storage circuit 110. The integration device 10 may automatically store/save, receive/aggregate, or consolidate/reorganize data (such as medical data), and may transmit the (received) data to another device (such as a host or a terminal). The medical data processed by the integration device 10 may be DICOM data objects in the DICOM format adopted by the PACS. The DICOM data objects include attribute data (namely, Attributes or DICOM tags), which may server as tags, and medical images. The attribute data may include attribute item(s) such as identification number(s), social security number(s), medical record number(s), date(s) of birth, or names, but not limited thereto. The attribute data may be used as an index to query/sort/search for medical data or to increase findability.

In short, different medical institutions may adopt different organizational architectures (namely, labeling methods, coding methods, numbering methods, or formats) for the attribute data (such as the medical record numbers). A medical record number, which serves as an attribute item, is organization specific and may be used as a patient identifier. In some embodiments, the organizational architecture for medical record number(s) of one medical institution may be coded/numbered with alphabet letter(s), number(s), label(s), or symbol(s), but is not limited thereto. In the present invention, when a DICOM data object is transferred, for example, from the medical institution Ha to the medical institution Hb, the integration device 10 may automatically edit, for example, a medical record number MR1 (also serve/referred to as a first attribute item) in the DICOM data object so as to convert the medical record number MR1 conforming to the organizational architecture of the medical institution Ha (also referred to as a first medical institution organizational architecture) into a medical record number MR2 (also serve/referred to as a second attribute item) conforming to the organizational architecture of the medical institution Hb (also referred to as a second medical institution organizational architecture), thereby efficiently integrate/reorganize medical data.

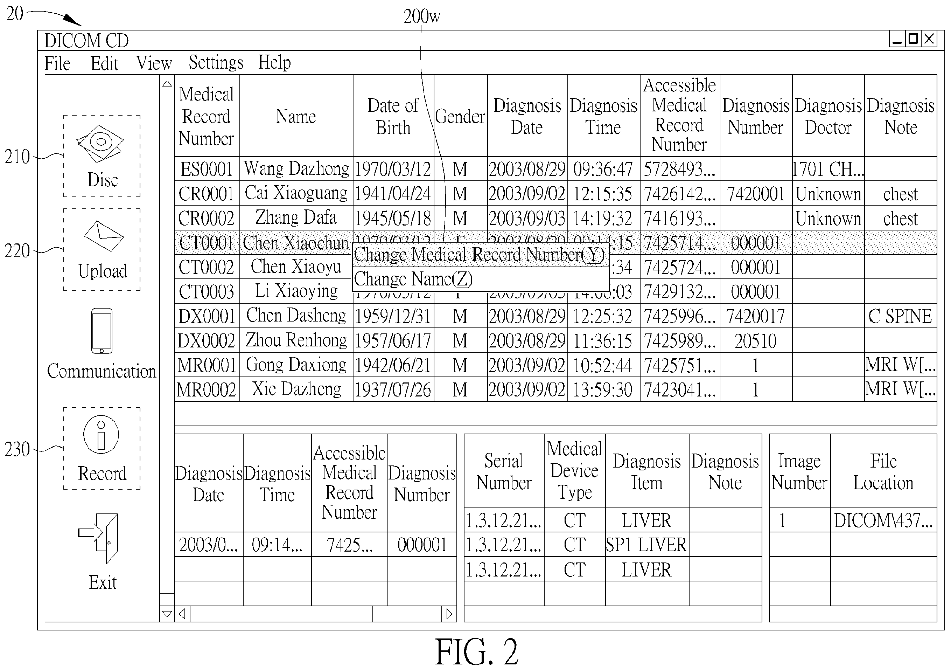

Specifically, a dedicated application software, such as a "ubiquitous diagnostic environment (UDE) application software", may be installed on the integration device 10. With the service of the DICOM worklist query, the work list of the attribute data is automatically read when the medical data is imported to the medical institution Hb. Therefore, after the integration device 10 receives a DICOM data object, the integration device 10 may learn/find that the DICOM data object includes the medical record number MR1 that complies with the organizational architecture of the medical institution Ha. The dedicated application software of the integration device 10 may search existing patient data/profiles or existing medical records for the medical record number (such as the medical record number MR2), which corresponds to the medical record number MR1 and complies with the organizational architecture of the medical institution Hb. The dedicated application software of the integration device 10 may compare the medical record number MR1 with the medical record number MR2 automatically. For instance, the organizational architecture of the medical institution Ha may be coded/numbered with alphabet letter(s) and number(s), and thus the medical record number MR1 may be CT0001 as shown in FIG. 2. For instance, the organizational architecture of the medical institution Hb may be coded/numbered with number(s) and symbol(s) (for example, dot), and thus the medical record number MR2 may be 7425714... as shown in FIG. 2. In this case, the data size (for example, the number of bits or the length of symbol(s)/letter(s)/character(s)/number(s)) of the organizational architecture of the medical institution Ha (or the data size of the medical record number MR1 for the medical institution Ha) is different from the data size of the organizational architecture of the medical institution Hb (or the data size of the medical record number MR2 for the medical institution Hb). Besides, number(s) comes after alphabet letter(s) in the organizational architecture of the medical institution Ha, while symbol(s) comes after number(s) in the organizational architecture of the medical institution Hb; that is to say, the order (for example, the numerical/alphabetical order) of the organizational architecture of the medical institution Ha (or the order of the medical record number MR1 for the medical institution Ha) is different from the order of the organizational architecture of the medical institution Hb (or the order of the medical record number MR2 for the medical institution Hb). When the medical record number MR1 is substantially different from the medical record number MR2, the integration device 10 may change the medical record number MR1 (in the DICOM data object) to the medical record number MR2 to integrate/reorganize medical data. In some embodiments, the existing patient data (such as the medical record number MR2) may be established/stored in the integration device 10 or another device (such as a host or a terminal) connected to the integration device 10 before the DICOM data object (including the medical record number MR1) is transmitted/input into the integration device 10.

"The medical record number MR1 corresponding to the medical record number MR2" means that "the medical record number MR1 and the medical record number MR2 correspond to the same identification number, date of birth, and/or name". In some embodiments, in order to search for the medical record number MR2, which corresponds to the medical record number MR1 and conforms to the organizational architecture of the medical institution Hb, the integration device 10 may use the identification number (in the DICOM data object which is newly input into the integration device 10) as a basis for comparison. If the same identification number which corresponds to the medical record number MR2 is found in the existing patient data, the integration device 10 may change the medical record number MR1 (in the DICOM data object) to the medical record number MR2. If the integration device 10 finds nothing (from the existing patient data) identical to the identification number (in the DICOM data object which is newly input into the integration device 10), the integration device 10 may select/search for other attribute items (such as name(s)), which are close/similar to the attribute items (in the DICOM data object which is newly input into the integration device 10) from the existing patient data. When the integration device 10 finds the (closest) attribute item, the user may edit the medical record number (such as the medical record number MR1) corresponding to the closest attribute item manually (to substitute the medical record number MR2 for the medical record number MR1); alternatively, the integration device 10 may automatically generate unused medical record number (also referred to as a third attribute item) to replace the medical record number MR1 using algorithms. In some embodiments, in order to search for the medical record number MR2, which corresponds to the medical record number MR1 and conforms to the organizational architecture of the medical institution Hb, the integration device 10 may use the date of birth and name (in the DICOM data object which is newly input into the integration device 10) as a basis for comparison. If the same name and date of birth which corresponds to the medical record number MR2 is found in the existing patient data, the integration device 10 may change the medical record number MR1 (in the DICOM data object) to the medical record number MR2. As set forth above, when the DICOM data object is transferred from the medical institution Ha to the medical institution Hb, the medical record number MR1 (in the DICOM data object) is modified to comply with the organizational architecture of the medical institution Hb so as to efficiently integrate/reorganize medical data.

In some embodiments, the DICOM data object may be stored/saved in an optical data storage device such as Compact Disc (CD) or digital versatile disc (DVD) but is not limited thereto. The integration device 10 is able to read the optical data storage device; for example, the integration device 10 may be equipped with an optical disc drive. The integration device 10 may automatically convert/replace the medical record number MR1 conforming to the organizational architecture of the medical institution Ha to/by the medical record number MR2 conforming to the organizational architecture of the medical institution Hb; alternatively, the user may work the dedicated application software of the integrated apparatus 10 to manually edit attribute item(s) (such as the medical record number MR1). For example, FIG. 2 is a schematic diagram of a user interface (UI) 20 of a dedicated application software of the integration device 10 shown in FIG. 1 according to an embodiment of the present invention. When the user selects (for example, right-clicks on the right mouse button of a mouse) certain attribute item, a window 200W of "Change Medical Record Number" and "Change Name" may pop up, which allows the user to replace the medical record number MR1 (in the DICOM data object) with the medical record number MR2 or to change the name in the DICOM data object.

In addition, the user may use/click/tap the function icon 210 of the user interface 20 to activate/burn (write data to) the optical disc in the optical disc drive; alternatively, the user may use/click/tap the function icon 220 to transmit/upload the edited medical data to the host (such as a DICOM server or a PACS server), a terminal or other devices. After the integration device 10 completes the transmission of the medical data, the user may use/click/tap the function icon 230 to query the transmission time, transmission status, operator, or transmission content. In some embodiments, the optical data storage device that stores DICOM data object is in compliance with to DICOM standards, and the DICOM data object may be located at the root level of the optical data storage device.

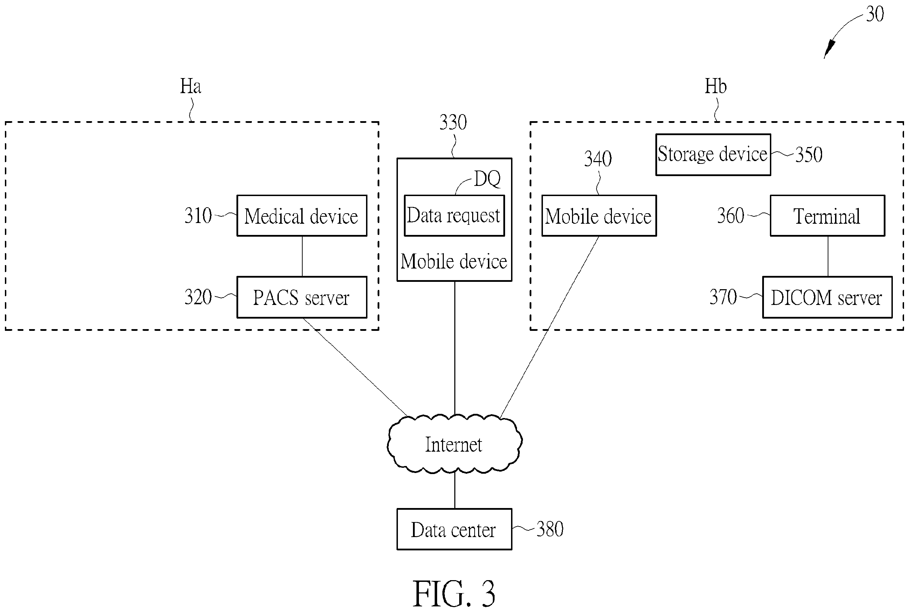

FIG. 3 is a schematic diagram of a data integration system 30 according to an embodiment of the present invention. The data integration system 30 may include a medical device 310, a PACS server 320, mobile devices 330, 340, a storage device 350, a (computer) terminal 360, a DICOM server 370 and a data center 380. The medical device 310 and the PACS server 320 may be (located/disposed) in the medical institution Ha; the mobile device 330 (also serve/referred to as a first mobile device) may be (located) in the medical institution Ha, or move from the medical institution Ha to the medical institution Hb, vice versa. The terminal 360 and the DICOM server 370 may be (located/disposed) in the medical institution Hb; the mobile device 340 (also serve/referred to as a second mobile device) and the storage device 350 may be (located) in the medical institution Hb. When the DICOM data object is to be transferred from the medical institution Ha to the medical institution Hb, the integration device 10 shown in FIG. 1 may be used/served as the mobile device 340 or the terminal 360 shown in FIG. 2 so as to integrate medical data, but is not limited thereto.

The medical device 310 may obtain/capture medical image(s) of a patient by means of photography or measurement, and the medical device 310 may transmit the medical image(s) to the PACS server 320. A medical image together with attribute data may be stored as a DICOM data object. It is noteworthy that the DICOM data object is only a standard for transmitting lossless image data. However, the present invention is not limited thereto, and the medical data (for example, the medical image(s) or attribute data) may be in a static digital image format such as GIF, JPG, or TIFF; alternatively, the medical data may be in a format which complies with Web access to the DICOM Persistent Object (WADO) standard. The PACS server 320 may store, obtain, transmit, and display the DICOM data object or medical data in a static digital image format to implement service(s) that a PACS system is able to provide.

The mobile device 330 or 340 may be a mobile phone, a tablet (computer), a computer/laptop, or other devices. In some embodiments, before the user of the mobile device 330 decide which medical data the user wants to transfer, the medical device 310 or the PACS server 320 uploads a plurality of medical data from the medical institution Ha to the data center 380 via the Internet. In some embodiments, the data center 380 may include a database server for storing medical data or a web server for supporting web services.

The mobile device 330 may issue service demand(s) to the data center 380 to indicate which medical data of the medical institution Ha the user intends to transfer. In some embodiments, in order to determine which medical data the user intends to transfer to the medical institution Hb, the user of the mobile device 330 may select from (all/some of) the medical data of the medical institution Ha by means of a dedicated application software or web browser application to transfer the selected medical data. The dedicated application software of the mobile device 330 may be a mobile application software (apps), such as the UDE application software. The user interface of the web browser application or the dedicated application software may present a compressed versions of the medical images or other related information such as the storage date(s) that the medical images are stored/saved, the diagnostic doctor(s) corresponding to the medical images, the diagnostic item(s)/categories corresponding to the medical images for the user to make decision. For example, the user may identify/figure out the selectable medical data by means of thumbnail(s)/preview(s) of the medical image(s), and the user may select one or more medical data from the selectable medical data. In some embodiments, before the user of mobile device 330 choose the medical data to be transferred via the Internet, the user is required to provide authentication/certification information such as the identification number, social security number, medical record number, or other account name, such that the user authentication/certification is performed before the user logs in. Once the user authentication/certification is passed, the data transmitted between the mobile device 330 and the data center 380 would be encrypted, for example, using Secure Sockets Layer (SSL) technology to encrypt. After the user of the mobile device 330 selects the medical data to be transferred, a data request DQ may be generated. In some embodiments, the data request DQ may be generated by the dedicated application software of the mobile device 330.

To inform the mobile device 340 which medical data the user of the mobile device 330 intends to transfer, the mobile device 330 may transfer the data request DQ to the mobile device 340. In some embodiments, the mobile device 330 may approach the mobile device 340 and display/present the data request DQ by presenting the image of the data request DQ for (the user of) the mobile device 340 to use. The data request DQ may include a uniform resource locator (URL), a Quick-Response code (QR code), a barcode, a token or a plain text. In some embodiments, the mobile device 330 may utilize radio frequency identification (RFID) or other kinds of Near-Field Communication (NFC) to transmit the data request DQ to the mobile device 340.

The mobile device 340 may download medical data from the Internet according to the data request DQ from the mobile device 330. For example, when the mobile device 330 displays/presents the data request DQ as a Quick-Response code, the mobile device 340 may approach the mobile device 330 to scan (the image of) the Quick-Response code and decode the Quick-Response code. Then, the mobile device 340 may access/download the medical data from the data center 380 via the Internet according to the data request DQ. In some embodiments, the mobile device 340 may scan the image with a dedicated application software, and download the medical data selected by the mobile device 330 to the mobile device 340. If the medical data is password protected or otherwise secured, the mobile device 340 may gain access by entering a key with its dedicated application software. The dedicated application software of the mobile device 340 may be a mobile application software, such as the UDE application software. That is to say, instead of sorting/searching for medical data, which correspond(s) to certain patient(s), one by one awkwardly using the identification number(s) of the patient(s), the mobile device 340 may obtain the medical data already selected/filtered by the user of the mobile device 330 all at once quickly/immediately according to the data request DQ of the mobile device 330 without using the identification number(s) of the patient(s) individually because the Quick-Response code serving as the data request DQ provides the exact path/link for the medical data already selected/filtered by the user of the mobile device 330. Besides, the mobile devices 330 needn't store/save the medical data selected by the user of the mobile device 330 substantially/physically (namely, it does not require the physical storage space of the mobile devices 330 for the medical data) but only needs to store/save the data request DQ which takes up less disk space. As a result, the convenience and efficiency the transfer of medical data is increased.

Since a local area network (LAN) of closed type may reduce the risk of medical data leakage/breach to maintain patient privacy, the medical institution Ha or Hb may be closed systems. In this case, the terminal 360 cannot access medical data from the data center 380 via a wide area network (WAN) such as the Internet. Thus, in some embodiments, the mobile device 340 may access the medical data from the data center 380, then the mobile device 340 may transmit the downloaded medical data to the storage device 350, and then the storage device 350 (for example, by means of C- STORE service) may transmit the medical data to the terminal 360. The storage device 350 may be a Subscriber Identity Module (SIM), Random-Access Memory (RAM), Read-Only Memory (ROM), flash memory, USB flash drive, hard disk, or optical data storage device, etc., but is not limited thereto.

The terminal 360 may be connected to the DICOM server 370 via wired or wireless communication, such as via a Bluetooth connection, a LAN connection or other wireless connections. The DICOM server 370 may store, obtain, transmit and display DICOM data object(s). The terminal 360 may transmit the consolidated medical data, such as the edited DICOM data object, to the DICOM server 370. The DICOM server 370 may parse the received DICOM data object, and store the attribute data of the DICOM data object in a specific database for other device to query. As set forth above, before the DICOM data object from the medical institution Ha is stored in the DICOM server 370 of the medical institution Hb, the medical record number MR1 of the DICOM data object is modified so as to comply with the organizational architecture of the medical institution Hb and hence may be systematically stored in the DICOM server 370.

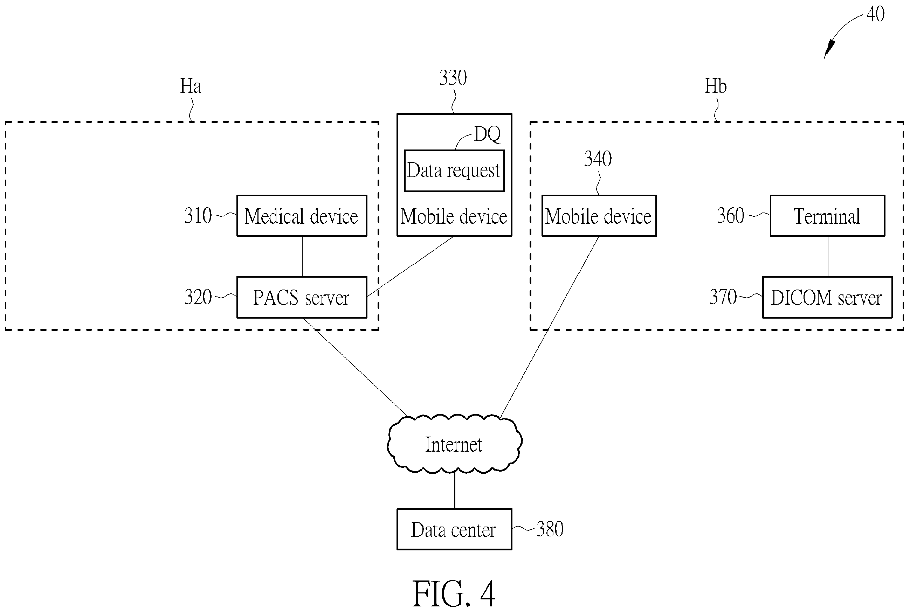

The data integration system 30 is an exemplary embodiment of the present invention, and those skilled in the art may readily make different substitutions and modifications. For example, please refer to FIG. 4. FIG. 4 is a schematic diagram of a data integration system 40 according to an embodiment of the present invention. The structure of the data integration system 40 is similar to that of the data integration system 30, and hence the same numerals and notations denote the same components in the following description.

Different from the data integration system 30, in the data integration system 40, in order to determine which medical data the user intends to transfer to the medical institution Hb, the mobile device 330 may be connected to the medical device 310 or the PACS server 320 via the LAN. The user of the mobile device 330 may choose the medical data of the medical institution Ha to be transferred through the user interface provided by its dedicated application software. The dedicated application software of the mobile device 330 may send a network transmission packet to the medical device 310 or the PACS server 320 according to the medical data selected by the user. The medical device 310 or the PACS server 320 may transmit another network transmission packet to the mobile device 330 after proper calculation. The PACS server 320 may correspondingly upload the medical data selected by the user to the data center 380 via the Internet. As a result, the dedicated application software for mobile device 330 may generate the data request DQ. In some embodiments, the network transmission packet is generated according to the Transmission Control Protocol (TCP) and the Internet Protocol (IP) protocol (namely, the TCP/IP protocol for short). In some embodiments, in order to ensure the security of the connection between the PACS server 320 and the data center 380, a secured encrypted virtual private network (VPN) technology may be adopted to allow only authorized communications.

In addition, when the terminal 360 cannot access the medical data from the data center 380 via the WAN, the mobile device 340 may first access the medical data from the data center 380, and then the mobile device 340 may transmit the downloaded medical data to the terminal 360. The mobile device 340 may be connected to the terminal 360 via wired or wireless communication, such as via a Bluetooth connection, a LAN connection or other wireless connections. Alternatively, the mobile device 340 may also be directly connected to the terminal 360 with a Universal Serial Bus (USB).

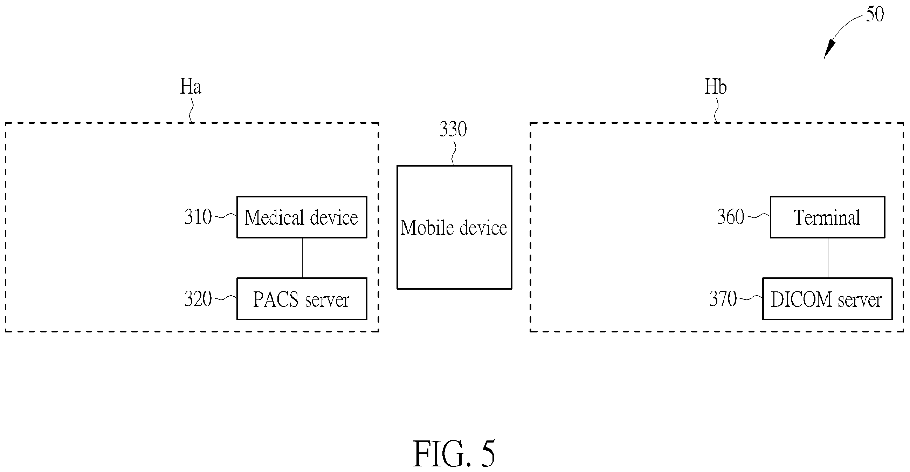

FIG. 5 is a schematic diagram of a data integration system 50 according to an embodiment of the present invention. In the data integration system 50, in order to directly obtain the medical data, the mobile device 330 may be connected to the medical device 310 via wired or wireless communication, such as via a Bluetooth connection, a LAN connection or other wireless connections. For example, when the user of mobile device 330 is breast photographed by the medical device 310 through mammography, the medical device 310 may not transmit the medical data to the PACS server 320 but only store the medical data in the medical device 310. The mobile device 330 may communicate directly with the medical device 310, and substantially/physically store/save the medical data. Similarly, in order to obtain the medical data directly, the mobile device 330 may also connect to the PACS server 320 through wired or wireless communication to substantially/physically store/save the medical data. For example, the user may select the medical data of the medical institution Ha by means of the user interface provided by the dedicated application software of the mobile device 330 to perform the transfer of the medical data. The dedicated application software may send a network transmission packet to the PACS server 320 according to the medical data selected by the user. The PACS server 320 may be transmit another network transmission packet to the mobile device 330 after calculation. As a result, the medical data may be transferred from the medical institution Ha to the medical institution Hb. Next, the mobile device 330 may be connected to the terminal 360 via wired or wireless communication so as to transmit the medical data to the terminal 360.

Alternatively, the mobile device 330 may also be directly connected to the medical device 310 or the terminal 360 with a Universal Serial Bus (USB), or a Digital Visual Interface, etc., to transfer the medical data from the medical institution Ha to the medical institution Hb. In this case, the mobile device 330 may replace with the storage device 350.

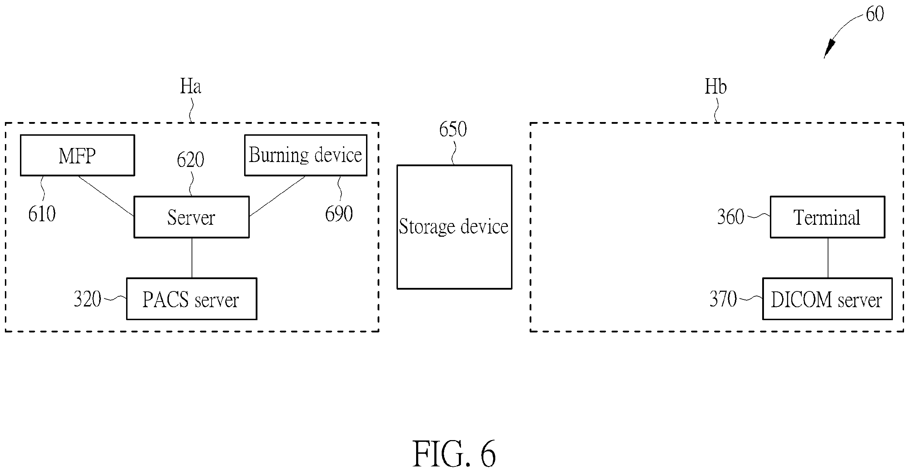

FIG. 6 is a schematic diagram of a data integration system 60 according to an embodiment of the present invention. Different from the data integration system 30, the data integration system 60 further includes a Multi-Function Product/Printer/Peripheral (MFP) 610, a server 620, a storage device 650 and a burning device 690. The MFP 610, the server 620, and the burning device 690 may be connected to each other or one another via wired or wireless communication, such as via a Bluetooth connection, a LAN connection or other wireless connections.

The MFP 610 is configured to provide a user interface, which may present a compressed versions of the medical images or other related information such as the storage date(s) that the medical images are stored/saved, the diagnostic doctor(s) corresponding to the medical images, the diagnostic item(s)/categories corresponding to the medical images, such that the user may identify/figure out the selectable medical data. Subsequently, the user may select one or more medical data from the selectable medical data to specify which medical data of the medical institution Ha is to be transferred. When the user issues a service request to the MFP 610, the MFP 610 may transmit a network transmit packet to the server 620. In some embodiments, a dedicated application software of the MFP 610 may communicate with a dedicated application software of the server 620 through the Application Programming Interface (API). Then, the server 620 may transmit a network transmission packet to request the PACS server 320 to provide the medical data selected by the user, and instruct the burning device 690 to store the medical data in the storage device 650. In some embodiments, the server 620 may receive medical data by means of WADO standard. The WADO standard may support the access of DICOM data object from HyperText Markup Language HTML (HTML) page(s) or Extensible Markup Language (XML) document(s). The burning device 690 may support multiple burning (for more than one storage device) or continuous burning. The malfunction state of the burning device 690 may be repaired by restarting the server 620; for example, the mechanism of the burning device 690 may be automatically bounced back. The storage device 650 may be an optical data storage device such as CD or DVD.

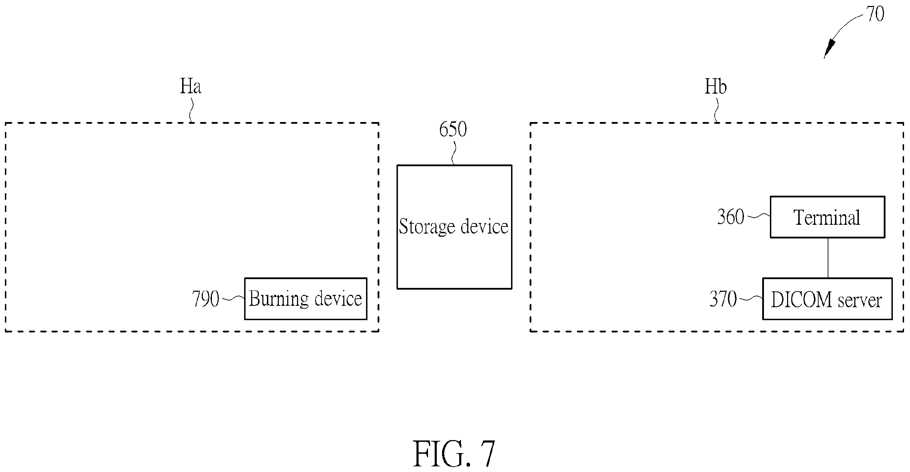

FIG. 7 is a schematic diagram of a data integration system 70 according to an embodiment of the present invention. Different from the data integration system 60, the data integration system 70 further includes a burning device 790. The burning device 790 may be a computer equipped with a burner/writer, but is not limited thereto. In addition to storing/writing medical data into the storage device 650, the burning device 790 may also store/write a dedicated application software (for instance, a DICOM viewer) into the storage device 650. By storing the dedicated application software, a DICOM data object may be read by the storage device 650 even if there is no dedicated application software installed in the medical institution Hb. In some embodiments, once the storage device 650 is connected to the terminal 360, the terminal 360 may automatically access the medical data of the storage device 650, and automatically transmit the medical data to the DICOM server 370 after the medical data is automatically edited.

In summary, a medical record number that complies with the organizational architecture of a medical institution may be converted into another medical record number that complies with the organizational architecture of another medical institution, such that the medical data always conforms to the current organizational architecture in the present invention (namely, the transferred medical data conforms to the organizational architecture of the latter while the medical data before the transfer conforms to the organizational architecture of the former) even if the two different medical institutions may adopt different organizational architectures, thereby efficiently integrate/reorganize medical data. In addition, the present invention provides multiple methods for the transfer of the medical data and the corresponding system architecture.

## Claims

1. A data integration system, **characterised by,** comprising:
an integration device (10), comprising:
a storage circuit (110), configured to store instructions of:
receiving a first digital imaging and communications in medicine, abbreviated to DICOM, data object comprising a first attribute item;
searching for a second attribute item corresponding to the first attribute item; and
converting the first attribute item of the first DICOM data obj ect to the second attribute item; and
a processing circuit (100), coupled to the storage device, configured to execute the instructions stored in the storage circuit.

2. The data integration system of claim 1, **characterised in that,** the first attribute item is a first medical record number conforming to a first medical institution organizational architecture, and the second attribute item is a second medical record number conforming to a second medical institution organizational architecture.

3. The data integration system of claim 1 or 2, **characterised in that,** the first attribute item and the second attribute item correspond to one identification number, date of birth, or name.

4. The data integration system of any of claims 1-3, **characterised in that,** the integration device automatically searches for the second attribute item corresponding to the first attribute item, and automatically converts the first attribute item of the first DICOM data object to the second attribute item.

5. The data integration system of any of claims 1-4, **characterised in that,** the integration device provides a user interface (20), the first attribute item of the first DICOM data object is changed to the second attribute item by manual editing.

6. The data integration system of any of claims 1-5, **characterised by,** further comprising:
a first mobile device (330), configured to generate a data request (DQ), the data request comprises one of a Uniform Resource Locator, a Quick-Response code, a barcode, a token, and a plain text; and
a second mobile device (340), configured to access the first DICOM data object via a wide area network, abbreviated to WAN, according to the data request.

7. The data integration system of claim 6, **characterised by,** further comprising:
a medical device (310); and
a picture archiving and communication system, abbreviated to PACS, server (320), wherein the first mobile device provides a first user interface, the first user interface is configured to indicate a plurality of DICOM data objects available for selection, the plurality of DICOM data objects come from the medical device or the PACS server, the first mobile device generates the data request when the first DICOM data object is selected from the plurality of DICOM data objects.

8. The data integration system of claim 7, **characterised by,** further comprising:
a database server, wherein the medical device or the PACS server transmits the plurality of DICOM data objects or merely transmits the first DICOM data object to the database server via the WAN

9. The data integration system of any of claims 1-8, **characterised by,** further comprising:
a multi-function product, abbreviated to MFP, (610), configured to provide a second user interface, the second user interface is configured to indicate a plurality of DICOM data objects available for selection, the plurality of DICOM data objects includes the first object DICOM data object;
a burning device (690);
a storage device (650), wherein the storage device is an optical data storage device; and
a server (620), coupled to the MFP, configured to instruct the burning device to store first DICOM data object selected from the plurality of DICOM data objects in the storage device.

10. The data integration system of any of claims 1-9, **characterised by,** further comprising:
a storage device (650), configured to store the first DICOM data object and a dedicated application software, and the dedicated application software supports reading of the first DICOM data object.

11. A data integration method, **characterized in that** the data integration method is configured to be executed by the data integration system of any of claims 1-10.
